# EUROPEAN PATENT SPECIFICATION

(11) **EP 2 292 214 B1**
(45) Date of publication and mention of the grant of the patent: **23.07.2014**
(21) Application number: 10251542.6
(22) Date of filing: 02.09.2010
(51) Int. Cl.: A61K 9/16, A61K 31/10, A61K 31/192

(54) **Highly absorbable drug composition and method of producing the same**
Stark absorbierbare Arzneimittelzusammensetzung und Verfahren zu deren Herstellung
Composition medicamenteuse hautement absorbable et son procédé de fabrication

(30) Priority: 02.09.2009 JP 2009202898
(43) Date of publication of application: 09.03.2011
(73) Proprietor: Toyo Sugar Refining Co., Ltd., Tokyo 1030016 (JP)
(72) Inventor: Tozuka, Yuichi, Gifu-shi Gifu 5028585 (JP); Uchiyama, Hiromasa, Gifu-shi Gifu 5028585 (JP); Takeuchi, Hirofumi, Gifu-shi Gifu 5028585 (JP); Iida, Yoshihisa, Ichihara-shi Chiba 2900046 (JP); Kometani, Takashi, Osaka-shi Osaka 5558502 (JP)
(74) Representative: Towler, Philip Dean

(56) References cited:
- JP-A- 8 268 855
- DATABASE WPI Week 200733 Thomson Scientific, London, GB; AN 2007-347067 XP002614345, & JP 2007 039419 A (TOYO SEITO KK) 15 February 2007 (2007-02-15)
- UCHIYAMA H ET AL: "Improvement of dissolution and absorption properties of poorly water-soluble drug by preparing spray-dried powders with alpha-glucosyl hesperidin", INTERNATIONAL JOURNAL OF PHARMACEUTICS, ELSEVIER BV, NL, vol. 392, no. 1-2, 15 June 2010 (2010-06-15), pages 101-106, XP027044509, ISSN: 0378-5173 [retrieved on 2010-03-20]

## Description

### BACKGROUND OF THE INVENTION

### Field of the invention

The present invention relates to a highly absorbable drug composition and a method of producing the composition and, more specifically, relates to a highly absorbable drug composition including a water-soluble compound (A) selected from an enzyme-treated hesperidin (α-glucosylated hesperetin glucoside), a stevia extract, or an enzyme-treated stevia (α-glucosylated steviol glucoside) and a poorly water-soluble drug (B) selected from flurbiprofen or probucol and relates to a method of producing the composition.

### Description of the Related Art

Recently, many novel drugs having useful pharmacological effects have been developed, but many of these novel drugs have a disadvantage of a poor absorbability. Administration of an excessive amount of a poorly absorbable drug in order to obtain a sufficient medicinal effect of the drug is undesirable from the viewpoints of side-effects and cost. In addition, even if a drug has certain absorbability, a further improvement in the absorbability can decrease the dosage amount thereof, which may further reduce the side-effects and the cost.

For example, flurbiprofen is a non-steroidal drug called NSAID and has antipyretic and analgesic activities, but also has side-effects such as gastric ulcer. Probucol is an agent used as an anti-hyperlipidemic drug, but also has side-effects such as rhabdomyolysis. Therefore, there has been a demand for a drug composition that exhibits a sufficient medicinal effect in a low dosage by improving the drug absorbability.

In patent document JP 2007-39419 A, the present applicant discloses that the solubility in water and the absorbability in vivo of naringin (flavonoid having physiological activities such as strengthening of capillaries) can be increased by means of a water-soluble composition containing naringin, α-glucosyl hesperidin, and β-monoglucosyl hesperetin. However, patent document JP 2007-39419 A does not disclose or suggest at all about improvement of solubilities or absorbabilities of compounds (drugs) other than naringin.

Furthermore, patent document JP 2007-63230 A discloses the finding that the absorbability of a poorly absorbable agent can be improved by preparing solid composite particles in which nanoparticles of a poorly absorbable agent are dispersed in a water-soluble filler by spray-drying the poorly absorbable agent having an average particle diameter of 0.01 to 2.0 µm and a composite particle having an average particle diameter of 0.5 to 50 µm and containing the water-soluble filler using a spray dryer or the like. As the water-soluble filler, sugar alcohols such as mannitol are mentioned, and as the poorly absorbable agent, flurbiprofen is mentioned.

### SUMMARY OF THE INVENTION

The present invention is intended to solve the problems occurring in the related art, and it is an object of the present invention to provide a drug composition that further improves absorbability of a poorly water-soluble drug selected from flurbiprofen or probucol and to provide a method of producing such a drug composition.

The present inventors have found that the solubility in water and the absorbability in vivo of each of flurbiprofen and probucol are increased by adding, for example, an enzyme-treated hesperidin, a stevia extract, or an enzyme-treated stevia to them, compared to those when the flurbiprofen and the probucol are each administered alone, and the present invention has been accomplished based on the above.

The highly absorbable drug composition of the present invention includes a water-soluble compound (A) selected from an enzyme-treated hesperidin, a stevia extract, or an enzyme-treated stevia; and a poorly water-soluble drug (B) selected from flurbiprofen or probucol.

The highly absorbable drug composition of the present invention is prepared by mixing an ethanol solution prepared by dissolving the poorly water-soluble drug (B) in ethanol and an aqueous solution prepared by dissolving the water-soluble compound (A) in water, and spray-drying the resulting mixed solution.

In the highly absorbable drug composition of the present invention, the enzyme-treated hesperidin is preferably an enzyme-treated hesperidin that is obtained by transferring sugar (glucose) from a glucosyl donor to hesperidin, hesperetin-7-glucoside, or hesperetin by the action of a glucosyltransferase.

In the highly absorbable drug composition of the present invention, the stevia extract is preferably a stevia extract that is obtained by extraction of stevia leaves and has, as a main ingredient, one or more steviol glucosides selected from the group consisting of stevioside, rebaudioside A, rebaudioside C, dulcoside A, rubusoside, steviolbioside, and rebaudioside B.

In the highly absorbable drug composition of the present invention, the enzyme-treated stevia is preferably an enzyme-treated stevia obtained by transferring sugar (glucose) from a glucosyl donor to one or more steviol glucosides selected from the group consisting of stevioside, rebaudioside A, rebaudioside C, dulcoside A, rubusoside, steviolbioside, and rebaudioside B by the action of a glucosyltransferase.

The method of producing a highly absorbable drug composition of the present invention includes mixing an ethanol solution prepared by dissolving a poorly water-soluble drug (B) selected from flurbiprofen or probucol in ethanol and an aqueous solution prepared by dissolving a water-soluble compound (A) selected from an enzyme-treated hesperidin, a stevia extract, or an enzyme-treated stevia in water; and spray-drying the resulting mixed solution.

According to the present invention, a highly absorbable drug composition where the absorbability of flurbiprofen or probucol, which are poorly water-soluble agents, is highly increased can be produced by a simple method. With such a highly absorbable drug composition, a sufficient medicinal effect can be exhibited by the administration of the poorly water-soluble drug in a low dosage. Therefore, even if the poorly water-soluble drug has side-effects, the side-effects can be reduced.

Furthermore, the present invention can increase the absorption rate of the poorly water-soluble agent and can show more immediate effect than ever. Therefore, the present invention can be applied to drugs that are required to rapidly show their medicinal effects.

Furthermore, the highly absorbable drug composition of the present invention also can improve the taste of the poorly water-soluble drug.

### BRIEF DESCRIPTION OF THE DRAWINGS

Fig. 1 is a graph showing flurbiprofen (FP) plasma concentration-time curves when untreated FP, FP (PM, αG-H) [drug composition prepared by physically mixing an enzyme-treated hesperidin (αG-H) and FP], and FP (SD, αG-H) [drug composition prepared by spray-drying αG-H and FP] were orally administered to rats;
Fig. 2 is a graph showing areas under flurbiprofen (FP) plasma concentration-time curves (AUC) of Fig. 1, when untreated FP, FP (PM, αG-H), and FP (SD, αG-H) were orally administered to rats;
Fig. 3 is a graph showing flurbiprofen (FP) plasma concentration-time curves when untreated FP, FP (PM, αG-Sweet) [drug composition prepared by physically mixing an enzyme-treated stevia (αG-Sweet) and FP], and FP (SD, αG-Sweet) [drug composition prepared by spray-drying αG-Sweet and FP] were orally administered to rats;
Fig. 4 is a graph showing areas under flurbiprofen (FP) plasma concentration-time curves (AUC) of Fig. 3, when untreated FP, FP (PM, αG-Sweet), and FP (SD, αG-Sweet) were orally administered to rats;
Fig. 5 is a graph showing probucol (PRO) plasma concentration-time curves when untreated PRO, PRO (SD, αG-H) [drug composition prepared by spray-drying αG-H and PRO], and PRO (SD, αG-Sweet) [drug composition prepared by spray-drying αG-Sweet and PRO] were orally administered to rats; and
Fig. 6 is a graph showing areas under probucol (PRO) plasma concentration-time curves (AUC) of Fig. 5, when untreated PRO, PRO (SD, αG-H), and PRO (SD, αG-Sweet) were orally administered to rats.

### DESCRIPTION OF THE PREFERRED EMBODIMENTS

The present invention will be specifically described below.

### [Highly absorbable drug composition]

The highly absorbable drug composition according to the present invention includes a water-soluble compound (A) selected from an enzyme-treated hesperidin, a stevia extract, or an enzyme-treated stevia and a poorly water-soluble drug (B) selected from flurbiprofen or probucol, as described below.

In the present invention, the highly absorbable drug composition can be modified according to the characteristics of the water-soluble compound (A) and the poorly water-soluble drug (B) that are used, but preferably, for example, includes the poorly water-soluble drug (B) in an amount of 5 to 20 parts by weight based on 100 parts by weight of the water-soluble compound (A).

The highly absorbable drug composition of the present invention is obtainable by mixing an ethanol solution prepared by dissolving the poorly water-soluble drug (B) in ethanol and an aqueous solution prepared by dissolving the water-soluble compound (A) in water, and spray-drying the resulting mixed solution.

In the present invention, the term "highly absorbable drug composition" refers to a drug composition having a higher absorbability than when the poorly water-soluble drug (B) contained in the composition is used alone. In this specification, the term "absorbability" means the total amount of a drug introduced into the body within a certain time after administration of the drug or a composition of the drug. Since the total amount of the drug introduced into the body is proportional to the area under the blood drug concentration-time curve (AUC), in the present invention (Examples shown below), the area under the blood drug concentration-time curve (AUC) is used as an indicator showing the absorbability.

Furthermore, in the present invention, the term "having immediate effects in absorption of a highly absorbable drug composition" means that the time before the blood drug concentration reaches a certain level is short. Note that the standard blood drug concentration varies depending on the type of the drug. In the case of flurbiprofen, for example, a concentration of 3.5 µg/mL or more can be used as the standard concentration, and in the case of probucol, for example, a concentration of 0.1 µg/mL or more can be used as the standard concentration.

### <Water-soluble compound (A)>

The water-soluble compound of the present invention is selected from an enzyme-treated hesperidin, a stevia extract, or an enzyme-treated stevia.

### • Enzyme-treated hesperidin (α-glucosyl hesperidin)

The enzyme-treated hesperidin (α-glucosyl hesperidin) in the present invention is a compound having one or more glucoses that are bonded to the glucosyl group in the rutinose unit of hesperidin by α1→4 bond(s), as shown in the following formula (I), and showing a solubility in neutral water of 10 g/100 g water or greater at ambient temperature, preferably 50 g/100 g water or greater. The compound in which only one glucose is bonded (n is 0 in the following formula (I)) is particularly called monoglucosyl hesperidin. 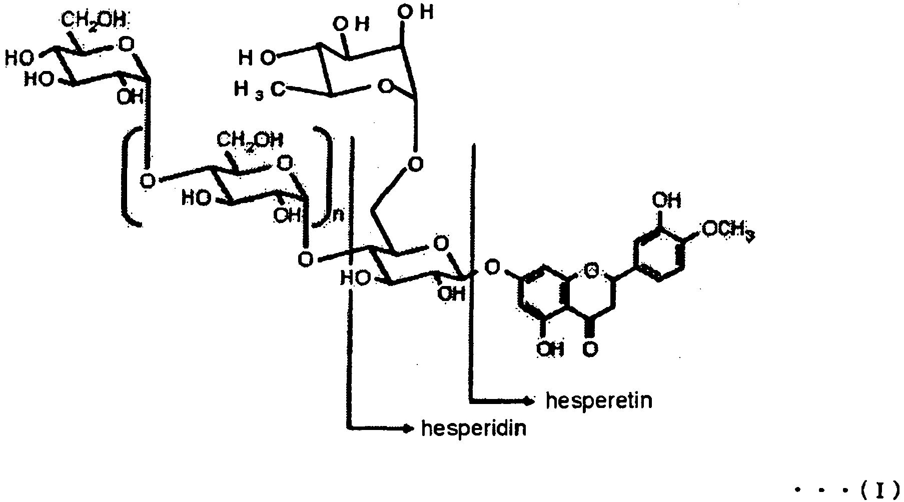 (in the formula (I), n represents 0 or an integer of 1 or more).

In the formula (I), hesperetin is the aglycone, and hesperidin is the poorly water-soluble flavonoid glucoside.

The enzyme-treated hesperidin is preferably an enzyme-treated hesperidin that is obtained by adding a glucosyl donor to hesperidin, hesperetin-7-glucoside, or hesperetin and transferring sugar (glucose) from the glucosyl donor to the hesperidin, hesperetin-7-glucoside, or hesperetin by the action of a glucosyltransferase.

For example, the enzyme-treated hesperidin is produced by allowing a glucosyl transferase, for example, cyclodextrin glucanotransferase (CGTase, EC 2.4.1.19) or another enzyme exhibiting a similar action, to react with hesperidin in the presence of α-glucosyl sugar compound (cyclodextrin, partially decomposed starch, or the like). With this enzyme treatment, one or a plurality of (about 2 to 20)glucoses binds to one molecule of hesperidin.

Furthermore, the monoglucosyl hesperidin can be produced by cutting the α1→4 bonded glucoses in α-glucosyl hesperidin having two or more glucoses so as to leave only a single glucose that is directly bonded to the glucosyl group in the rutinose unit by reacting a sugar hydrolase, for example, glucoamylase (EC 3.2.1.3) or another enzyme exhibiting a similar action, with the α-glucosyl hesperidin.

Incidentally, the enzyme-treated hesperidin is usually a mass of enzyme-treated hesperidins having different numbers of glucoses, that is, a mass composed of monoglucosyl hesperidin and other enzyme-treated hesperidins. Furthermore, since the enzyme-treated hesperidin is generally produced by such enzyme treatment described above, the enzyme-treated hesperidin is a mixture containing unreacted hesperidin and other derivatives.

The effects, such as an improvement in the water solubility, of the present invention can be exerted regardless whether the enzyme-treated hesperidin is monoglucosyl hesperidin or others. The ratio of the monoglucosyl hesperidin in the α-glucosyl hesperidin can be controlled by a method known to those skilled in the art, for example, by changing conditions (such as reaction time) of enzyme treatment with the above-mentioned glucoamylase.

The enzyme-treated hesperidin that can be used in the present invention may be commercially available one, such as "αG hesperidin H" and "αG hesperidin PA" (manufactured by Toyo Sugar Refining Co., Ltd., and available from Ezaki Glico Co., Ltd.).

### • Stevia extract

The stevia extract in the present invention includes a steviol glucoside obtained by extraction of stevia (*Stevia rebaudiana Bertoni*) leaves as a main ingredient at an amount of 80% or more in its dried state. In this specification, the term "steviol glucoside" means one or more glucosides selected from the group consisting of stevioside, rebaudioside A, rebaudioside C, dulcoside A, rubusoside, steviolbioside, and rebaudioside B.

The stevia extract may be used alone or in a form of liquid, powder, granules, or the like including the stevia extract and a diluent, a filler, or the like.

The stevia extract that can be used in the present invention may be commercially available one, such as "Stevilose 90", "Stevilose 90A", and "Stevilose 50" (manufactured by Toyo Sugar Refining Co., Ltd.).

### • Enzyme-treated stevia

The enzyme-treated stevia in the present invention is preferably an enzyme-treated stevia that is obtained by adding a glucosyl donor to one or more steviol glucosides selected from the group consisting of stevioside, rebaudioside A, rebaudioside C, dulcoside A, rubusoside, steviolbioside, and rebaudioside B and transferring sugar (glucose) from the glucosyl donor to the steviol glucoside by the action of glucosyltransferase. For example, the enzyme-treated stevia is obtained by adding glucose to a stevia extract using α-glucosyltransferase or the like. Such an enzyme-treated stevia has a solubility in neutral water of 2000 g/L water or greater at ambient temperature. This enzyme-treated stevia in its dried state contains 80.0% (wt%) or more of steviol glucosides as the total amount of α-glucosyl steviol glucosides and unreacted steviol glucosides and 65.0% or more of α-glucosyl steviol glucosides. Since the enzyme-treated stevia generally contains unreacted stevia, the enzyme-treated stevia is a mixture of the stevia extract and substances in which glucose is added to the stevia extract.

The enzyme-treated stevia may be used alone or in a form of liquid, powder, granules, or the like of the enzyme-treated stevia diluted with a diluent, a filler, or the like.

The enzyme-treated stevia used in the present invention may be commercially available one, such as "αG Sweet PX", "αG Sweet P", "αG Sweet PA", and "αG Sweet H" (manufactured by Toyo Sugar Refining Co., Ltd.).

### <Poorly water-soluble drug (B)>

In the present invention, the poorly water-soluble drug (B) is flurbiprofen or probucol.

The flurbiprofen (hereinafter, also referred to as "FP") is a drug having efficacy against pain, inflammatory, chronic rheumatism, osteoarthritis, lumbago, pulpitis, and pericementitis and causing peptic ulcer, such as gastric ulcer, as side-effects. The probucol (hereinafter, also referred to as "PRO") is an anti-hyperlipidemic drug and causes cardiac dysrhythmia or rhabdomyolysis as side-effects.

The flurbiprofen (FP) is a drug classified in Class II in the Biopharmaceutics Classification System (BCS), which is a system in the USA, and is a drug that is poor in solubility but has a high absorbability (the solubility in neutral water is 25 µg/mL water at 37°C). Therefore, in order to further improve the absorbability of FP, it is suggested to improve the absorbability by increasing the rate of dissolution and the solubility of FP. Thus, since sufficient medicinal effects can be exerted even when FP is administered at a low dosage by improving the absorbability of FP, it is possible to reduce the side-effects such as gastric ulcer.

The probucol (PRO) is not classified in the BCS, but is an agent having a very low bioavailability to human: about 5%. Therefore, it is necessary to be administered twice a day at a dose of 250 mg per one time. Thus, the necessary ingestion dose is high. It is considered that a factor of the low bioavailability is the very low solubility in neutral water of probucol: 3 to 5 ng/mL water at 37°C. Accordingly, it can be expected that the bioavailability can be improved by increasing the solubility of probucol and thereby that the amount of administration can be decreased to thereby reduce the side-effects.

These poorly water-soluble drugs (B) are both highly bitter drugs, but it is thought that the addition of the water-soluble compound (A) of the present invention can mask the bitterness, reducing the uncomfortable taste.

In addition, it is suggested that indomethasine, ketoprofen, and piroxicam, which are analgesics, and statin, which is an anti-hyperlipidemic drug, can be improved in their absorbability by being mixed with the water-soluble compound (A).

### [Method of producing highly absorbable drug composition]

The highly absorbable drug composition of the present invention is produced by mixing one or more water-soluble compounds (A) selected from an enzyme-treated hesperidin, a stevia extract, or an enzyme-treated stevia and a poorly water-soluble drug (B) selected from flurbiprofen or probucol.

The method of the production is spray-drying that can produce highly-absorbable granular (spherical) drug composition by spraying a mixed solution of an aqueous solution of the water-soluble compound (A) and an ethanol solution of the poorly water-soluble drug (B) to the inside of a chamber from a nozzle and bringing the mixed solution into contact with blasts of hot air in the chamber for instant drying. When the highly absorbable drug composition thus-obtained by the spray-drying has an average particle diameter of 1 to 10 µm, preferably 2 to 5 µm, the poorly water-soluble drug contained the composition is excellent in solubility in water and absorbability in vivo. In addition, the spray-drying has advantages that drying time is short, which is advantageous for relatively heat-sensitive drugs, and that continuous high-volume production is possible, which enables to produce the highly absorbable drug composition at low cost.

The highly absorbable drug composition is produced by spray-drying. A common spray dryer (for example, Model "GS31", a product of Yamato Scientific Co., Ltd.) can be used. Various conditions for the spray-drying can be suitably controlled while considering, for example, the particle diameter of particles to be produced. For example, the concentration of an aqueous solution of the water-soluble compound (A), the concentration of a solution (preferably, ethanol solution) of the poorly water-soluble drug (B), and the mixture ratio of these two solutions may be suitably controlled according to the solubilities of the water-soluble compound (A) and the poorly water-soluble drug (B), the ratios of the water-soluble compound (A) and the poorly water-soluble drug (B) to a highly absorbable drug composition to be produced, and so on. The ethanol concentration (in Examples described below, about 60% or 80%) in a mixed solution used in the spray-drying is controlled in a relatively higher range than that (20%) of a solution used in usual spray-drying. In addition, the nozzle diameter of a spray dryer is usually 0.2 to 1.3 mm, the spray rate of the mixed solution to the inside of a chamber is usually 5 to 20 mL/min, the inlet temperature and the outlet temperature of hot air in a chamber are usually 100 to 160°C and 40 to 80°C, respectively, and the pressure is 0.1 to 0.15 MPa.

Examples

Preferred embodiments of the present invention will be described more specifically based on examples below.

### [Preparation of samples]

### · Preparation Example 1 (preparation of drug compositions A and B)

Five grams of an enzyme-treated hesperidin (αG hesperidin PA-T, manufactured by Toyo Sugar Refining Co., Ltd., and available from Ezaki Glico Co., Ltd.) was dissolved in 40 mL of distilled water to prepare an aqueous solution, and 0.5 g of a poorly water-soluble drug (flurbiprofen (FP) or probucol (PRO)) was dissolved in 160 mL of ethanol to prepare an ethanol solution. The aqueous solution and the ethanol solution were mixed, and the resulting mixture was spray-dried with a spray dryer (Model "GS31", a product of Yamato Scientific Co., Ltd., inlet temperature: 120°C, outlet temperature: 50 to 60°C, flow rate: 10 mL/min, pressure: 0.13 MPa) to obtain a drug composition A containing flurbiprofen or a drug composition B containing probucol.

### · Comparative Preparation Example 2 (preparation of drug composition C)

Five grams of an enzyme-treated hesperidin (αG hesperidin PA-T) and 0.5 g of a poorly water-soluble drug (flurbiprofen) were mixed (physical mixing) in a mortar to obtain a drug composition C containing flurbiprofen.

### • Preparation Example 3 (drug compositions D and E)

Five grams of an enzyme-treated stevia (αG Sweet PX, manufactured by and available from Toyo Sugar Refining Co., Ltd.) was dissolved in 80 mL of distilled water to prepare an aqueous solution, and 0.5 g of a poorly water-soluble drug (flurbiprofen or probucol) was dissolved in 120 mL of ethanol to prepare an ethanol solution. The aqueous solution and the ethanol solution were mixed, and the resulting mixture was spray-dried with a spray dryer (Model "GS31", a product of Yamato Scientific Co., Ltd., inlet temperature: 140°C, outlet temperature: 60 to 70°C, flow rate: 10 mL/min, pressure: 0.13 MPa) to obtain a drug composition D containing flurbiprofen or a drug composition E containing probucol.

### · Comparative Preparation Example 4 (drug composition F)

Five grams of an enzyme-treated stevia (αG Sweet PX) and 0.5 g of a poorly water-soluble drug (flurbiprofen) were physically mixed to obtain a drug composition F containing flurbiprofen.

### [Administration of prepared drugs]

As test animals, 9-week-old male clean wistar rats (five rats for each of drug compositions A to F) were used.

The above-prepared drug compositions A, C, D, and F were each suspended in distilled water so that the concentration of the drug composition was 0.5 mg/mL, and the drug compositions B and E were each suspended in distilled water so that the concentration of the drug composition was 50 mg/mL.

The suspension containing the drug composition A, C, D, or F was directly administered to the stomach of the clean wistar rats using an oral sonde (Fuchigami Kikai) at a dose of 2 mg/kg, and the suspension containing the drug composition B or E was similarly administered at a dose of 200 mg/kg.

After the administration, blood was collected from the neck vein of each clean wistar rat at predetermined time intervals and was centrifuged (10000 rpm, 5 min) to obtain plasma.

### [Quantitative measurement of blood drug concentration]

Four hundred microliters of methanol was added to 100 µL of the obtained plasma, and the mixture was mixed for 1 minute with a vortex mixer and was centrifuged (10000 rpm, 5 min). Then, 300 µL of the supernatant was collected and was dried under reduced pressure for a half day. One hundred microliters of ethanol was added to the resulting dried substance, and the blood drug concentration was measured using a UV detector (JASCO PU-970) of HPLC (JASCO PU-980).

The HPLC conditions for quantitative measure of drug amounts absorbed by the clean wistar rats administered with the drug composition A, C, D, or F were absorption wavelength: 254 nm, flow rate: 0.7 min, column temperature: 40°C, column: 5C₁₈-MSII (4.6x150 mm), and mobile phase: methanol/water/1 M acetic acid = 80/20/1. The HPLC conditions for quantitative measure of drug amounts absorbed by the clean wistar rats administered with the drug composition B or E were absorption wavelength: 242 nm, flow rate: 1.0 min, column temperature: 50°C, column: 5C₁₈-MSII (4.6x150 mm), and mobile phase: acetonitrile/water = 90/10.

### [Results]

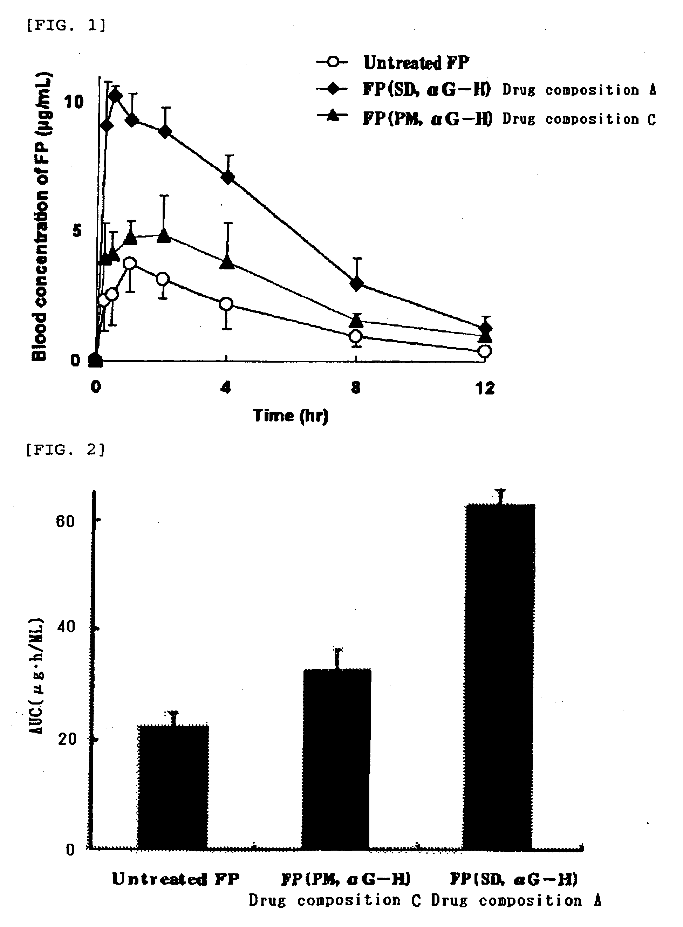

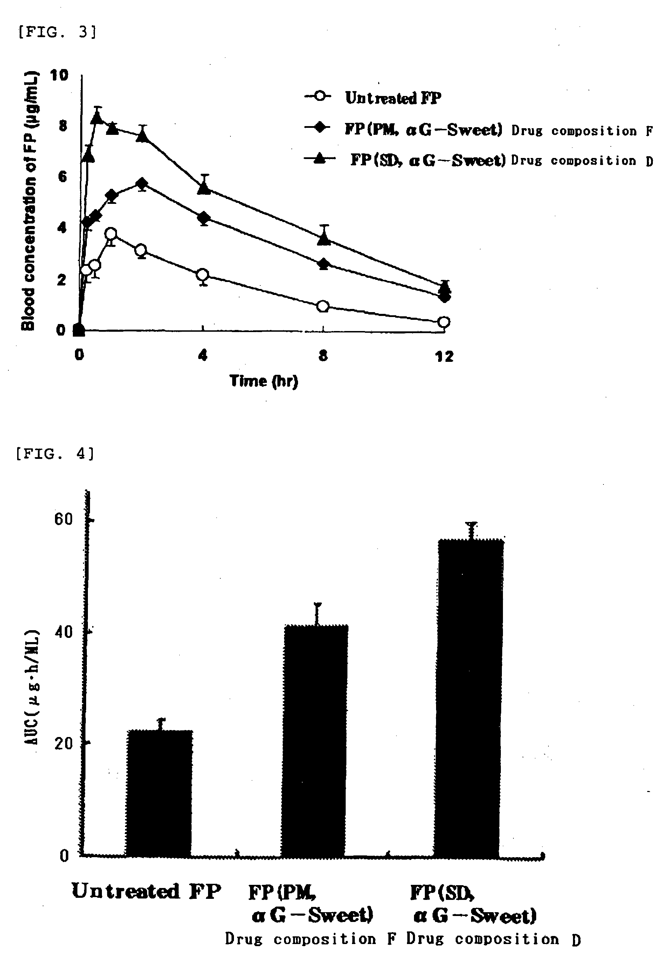

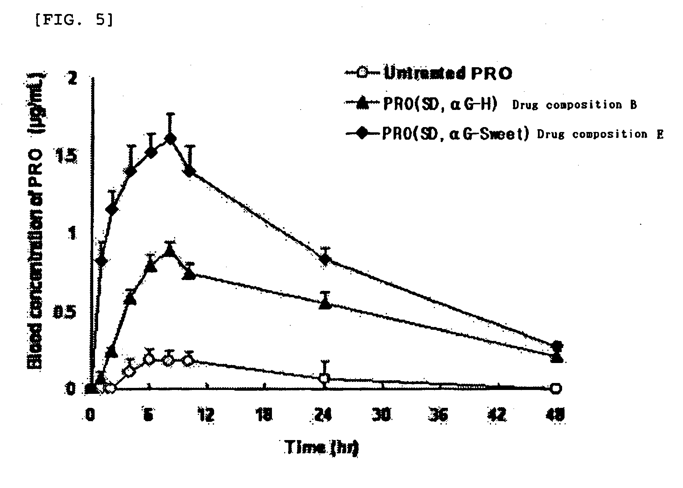

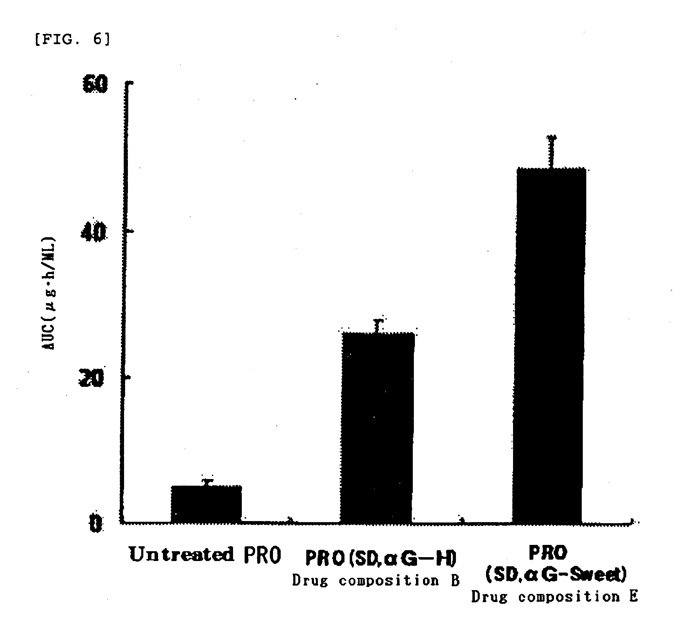

Based on the thus-measured blood drug concentrations of the drug compositions, concentration changes with passage of time and areas under blood drug concentration-time curves (AUC) were studied. The results of the drug compositions A and C are shown in Table 1 and Figs. 1 and 2, the results of the drug compositions D and F are shown in Table 1 and Figs. 3 and 4, and the results of the drug compositions B and E are shown in Table 1 and Figs. 5 and 6.

Note that the values of AUC and blood drug concentration in Table 1 and Figs. 1 to 6 are averages in the five clean wistar rats administered with each of the drug compositions A to F, and "SE" means standard error.

The "untreated FP" shows the results measured as above except that FP was used alone (without mixing with αG-H or αG-Sweet) as a control, and the "untreated PRO" shows the results measured as above except that PRO was used alone (without mixing with αG-H or αG-Sweet) as a control.

The "αG-H" means the enzyme-treated hesperidin (αG hesperidin PA-T), and the "αG-Sweet" means the enzyme-treated stevia (αG Sweet PX). The "PM" means the drug composition produced by physical mixing, and the "SD" means the drug composition produced by spray-drying. That is, FP (PM, αG-H) means the drug composition prepared by physically mixing the enzyme-treated hesperidin and flurbiprofen, and FP (SD, αG-H) means the drug composition prepared by spray-drying the enzyme-treated hesperidin and flurbiprofen.

It was confirmed from the ratio of the AUC of each drug composition to the AUC of the control (Table 1) that the absorbabilities of FP and PRO were improved in every drug composition and confirmed from the results of FP that the effect of improving absorbability by SD (drug compositions A and D) was higher than that by PM (drug compositions C and F). Furthermore, the peak values of the blood drug concentrations of FP (Figs. 1 and 3) and PRO (Fig. 5) were notably increased compared to those of the control. The time before the blood drug concentration of FP of each of the drug compositions A, C, D, and F reaches 3.5 µg/mL and the time before the blood drug concentration of PRO of each of the drug compositions B and E reaches 0.1 µg/mL were shortened, compared to those of the controls (Figs. 1, 3, and 5). That is, the drug composition of the present invention has immediate effects in absorption of poorly water-soluble drugs compared to those when the poorly water-soluble drugs are each used alone.

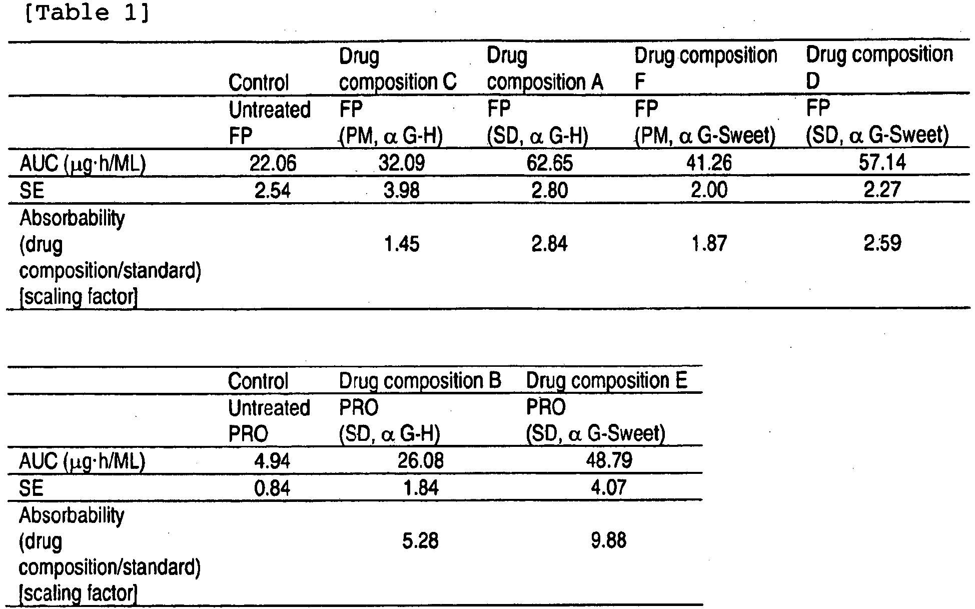

## Claims

1. A product obtainable by mixing an ethanol solution prepared by dissolving a poorly water-soluble drug (B) selected from flurbiprofen or probucol in ethanol and an aqueous solution prepared by dissolving a water-soluble compound (A) selected from an enzyme-treated hesperidin, a stevia extract, or an enzyme-treated stevia in water, and spray-drying the resulting mixed solution.

2. The product according to claim 1, wherein the amount of the poorly water-soluble drug (B) is 5 to 20 parts by weight based on 100 parts by weight of the water-soluble compound (A).

3. The product according to Claim 1 or 2, wherein the enzyme-treated hesperidin is an enzyme-treated hesperidin that is obtained by adding a glucosyl donor to hesperidin, hesperetin-7-glucoside, or hesperetin and transferring sugar (glucose) from the glucosyl donor to the hesperidin, hesperetin-7-glucoside, or hesperetin by the action of a glucosyltransferase.

4. The product according to Claim 1 or 2, wherein the stevia extract is a stevia extract that is obtained by extraction of stevia leaves and has, as a main ingredient, one or more steviol glucosides selected from the group consisting of stevioside, rebaudioside A, rebaudioside C, dulcoside A, rubusoside, steviolbioside, and rebaudioside B.

5. The product according to Claim 1 or 2, wherein the enzyme-treated stevia is an enzyme-treated stevia that is obtained by adding a glucosyl donor to one or more steviol glucosides selected from the group consisting of stevioside, rebaudioside A, rebaudioside C, dulcoside A, rubusoside, steviolbioside, and rebaudioside B and transferring sugar (glucose) from the glucosyl donor to the steviol glucoside by the action of a glucosyltransferase.

6. A method of producing a highly absorbable drug composition, comprising:
mixing an ethanol solution prepared by dissolving a poorly water-soluble drug (B) selected from flurbiprofen or probucol in ethanol and an aqueous solution prepared by dissolving a water-soluble compound (A) selected from an enzyme-treated hesperidin, a stevia extract, or an enzyme-treated stevia in water; and
spray-drying the resulting mixed solution.

## Patentansprüche

1. Produkt, das durch Mischen einer Ethanollösung, die durch Lösen eines schlecht wasserlöslichen Arzneimittels (B) ausgewählt unter Flurbiprofen oder Probucol in Ethanol hergestellt wird, und einer wässrigen Lösung, die durch Lösen einer wasserlöslichen Verbindung (A) ausgewählt unter einem enzymbehandelten Hesperidin, einem Steviaextrakt oder einer enzymbehandelten Stevia in Wasser hergestellt wird, und Sprühtrocknen der resultierenden gemischten Lösung erhältlich ist.

2. Produkt nach Anspruch 1, wobei die Menge des schlecht wasserlöslichen Arzneimittels (B) 5 bis 20 Gewichtsteile, auf 100 Gewichtsteile der wasserlöslichen Verbindung (A) bezogen, beträgt.

3. Produkt nach Anspruch 1 oder 2, wobei das enzymbehandelte Hesperidin ein enzymbehandeltes Hesperidin ist, das durch Hinzugeben eines Glucosyldonors zu Hesperidin, Hesperetin-7-glucosid oder Hesperetin und Übertragen von Zucker (Glucose) von dem Glucosyldonor zu dem Hesperidin, Hesperetin-7-glucosid oder Hesperetin durch die Wirkung einer Glucosyltransferase erhalten wird.

4. Produkt nach Anspruch 1 oder 2, wobei das Steviaextrakt ein Steviaexterakt ist, das durch Extraktion von Steviablättern erhalten wird und als Hauptinhaltsstoff ein oder mehrere Steviolglucoside aufweist, ausgewählt aus der Gruppe bestehend aus Steviosid, Rebaudiosid A, Rebaudiosid C, Dulcosid A, Rubusosid, Steviolbiosid und Rebaudiosid B.

5. Produkt nach Anspruch 1 oder 2, wobei die enzymbehandelte Stevia eine enzymbehandelte Stevia ist, die durch Hinzugeben eines Glucosyldonors zu einem oder mehreren Steviolglucosiden ausgewählt aus der Gruppe bestehend aus Steviosid, Rebaudiosid A, Rebaudiosid C, Dulcosid A, Rubusosid, Steviolbiosid und Rebaudiosid B und Übertragen von Zucker (Glucose) von dem Glucosyldonor zu dem Steviolglucosid durch die Wirkung einer Glucosyltransferase erhalten wird.

6. Verfahren zur Herstellung einer hochabsorbierbaren Arzneimittelzusammensetzung, umfassend:
das Mischen einer Ethanollösung, die durch Lösen eines schlecht wasserlöslichen Arzneimittels (B) ausgewählt unter Flurbiprofen oder Probucol in Ethanol hergestellt wird, und einer wässrigen Lösung, die durch Lösen einer wasserlöslichen Verbindung (A) ausgewählt unter einem enzymbehandelten Hesperidin, einem Steviaextrakt oder einer enzymbehandelten Stevia in Wasser hergestellt wird; und
das Sprühtrocknen der resultierenden gemischten Lösung.

## Revendications

1. Produit qui peut être obtenu en mélangeant une solution éthanolique préparée en dissolvant un médicament médiocrement soluble dans l'eau (B) choisi entre le flurbiprofène et le probucol dans de l'éthanol et une solution aqueuse préparée en dissolvant un composé soluble dans l'eau (A) choisi entre une hespéridine traitée par un enzyme, un extrait de stévie et une stévie traitée par un enzyme dans de l'eau, et en séchant par pulvérisation la solution mixte obtenue.

2. Produit selon la revendication 1, dans lequel la quantité du médicament médiocrement soluble dans l'eau (B) est de 5 à 20 parties en poids sur la base de 100 parties en poids du composé soluble dans l'eau (A).

3. Produit selon la revendication 1 ou la revendication 2, dans lequel l'hespéridine traitée par un enzyme est une hespéridine traitée par un enzyme qui est obtenue en ajoutant un donneur de glucosyle à de l'hespéridine, à un hespérétine-7-glucoside ou à de l'hespérétine et en transférant du sucre (glucose) du donneur de glucosyle à l'hespéridine, à l'hespérétine-7-glucoside ou à l'hespérétine par l'action d'une glucosyltransférase.

4. Produit selon la revendication 1 ou la revendication 2, dans lequel l'extrait de stévie est un extrait de stévie qui est obtenu par extraction de feuilles de stévie et présente, comme ingrédient principal, un ou plusieurs glucosides de stéviol choisis dans le groupe constitué du stévioside, du rebaudioside A, du rebaudioside C, du dulcoside A, du rubososide, du stéviolbioside et du rebaudioside B.

5. Produit selon la revendication 1 ou la revendication 2, dans lequel la stévie traitée par un enzyme est une stévie traitée par un enzyme qui est obtenue par addition d'un donneur de glucosyle à un ou plusieurs glucosides de stéviol choisis dans le groupe constitué du stévioside, du rebaudioside A, du rebaudioside C, du dulcoside A, du rubososide, du stéviolbioside et du rebaudioside B et en transférant du sucre (glucose) du donneur de glucosyle au glucoside de stéviol par l'action d'une glucosyltransférase.

6. Procédé de production d'une composition médicamenteuse fortement absorbable, comprenant :
le mélange d'une solution éthanolique préparée en dissolvant un médicament médiocrement soluble dans l'eau (B) choisi entre le flurbiprofène et le probucol dans de l'éthanol et d'une solution aqueuse préparée en dissolvant un composé soluble dans l'eau (A) choisi entre une hespéridine traitée par un enzyme, un extrait de stévie et une stévie traitée par un enzyme dans de l'eau, et
le séchage par pulvérisation de la solution mixte obtenue.
